# EUROPEAN PATENT SPECIFICATION

(11) **EP 2 393 489 B1**
(45) Date of publication and mention of the grant of the patent: **08.10.2014**
(21) Application number: 10703804.4
(22) Date of filing: 03.02.2010
(51) Int. Cl.: A61K 31/165, A61K 9/26, A61K 9/20, A61K 31/4985

(54) **Moisture-activated granulation process**
Flüssigkeitsaktiviertes Granulationsverfahren
Processus de granulation à humidité activée

(30) Priority: 05.02.2009 EP 09001641; 16.12.2009 EP 09015553
(43) Date of publication of application: 14.12.2011
(73) Proprietor: Krka, tovarna zdravil, d.d., Novo mesto, 8000 Novo Mesto (SI)
(72) Inventor: STUKELJ, Mitja, 8222 Otocec (SI); SKRABANJA, Vida, 8000 Novo mesto (SI); FERLAN, Andrej, 1275 Smartno Pri Litiji (SI); VRECER, Franc, 8351 Straza pri Novem mestu (SI); KUKEC, Simon, 1000 Ljubljana (SI)
(74) Representative: UEXKÜLL & STOLBERG
(86) International application number: PCT/EP2010/000667
(87) International publication number: WO 2010/089105

(56) References cited:
- EP-A1- 1 985 310
- WO-A-2006/041974
- US-A1- 2006 018 960
- Australian Goverment - Therapeutic Goods Administration: "Australian regulatory guidelines for OTC medicines", , 1 July 2003 (2003-07-01), pages COVER-4A.3, XP002587617, Retrieved from the Internet: URL:www.tga.gov.au/docs/pdf/argom_4.pdf [retrieved on 2010-06-17]

## Description

The present invention relates to a moisture-activated granulation process for manufacturing pharmaceutical compositions, in particular solid dosage forms of active substances which are prone to chemical degradation and/or physical phase transitions upon contact with water or aqueous liquids such as those used during conventional wet granulation processes. The present invention also relates to the pharmaceutical formulations and dosage forms obtained from the process according to the invention and their use as medicaments.

### Background of the Invention

Numerous active substances are sensitive to the presence of moisture. This can pose significant problems in the manufacture of pharmaceutical formulations and dosage forms containing such active substances. Moisture may stem from the excipients used in the formulation or from the manufacturing process, e.g. aqueous granulation.

The presence of moisture is in particular undesirable if the active substance is prone to chemical degradation and/or physical phase transitions into an undesired crystalline and/or amorphous form (polymorphism) when being in contact with water or water-containing solutions. Examples for such active substances are aliskiren, in particular aliskiren hemifumarate, ACE inhibitors selected from lisinopril, ramipril, enalapril, teophiline, valsartan, orlistat, desloratidine, solifenacin and its salts such as the maleate, malonate, hydrogensulphate, succinate and citrate, donepezil and its salts, benzimidazole proton pump inhibitors selected from omeprazole, esomeprazole, rabeprazole, pantoprazole, lansoprazole and their salts such as sodium and magnesium salts, inhibitors of HGMCo reductase selected from rosuvastatin, atorvastatin and its salts, in particular atorvastatin Ca, fluvastatin and its salts, platelet aggregation inhibitors selected from clopidrogel, in particular clopidogrel salts with HCl and H₂SO₄, and prasugrel. Said active substances are in particular known to form chemical degradation products when moisture is present in the pharmaceutical formulation containing them. Another example is tadalafil, a phosphodiesterase type 5 (PDE5) inhibitor.

Other active substances such as teophiline, pantoprazole, aliskiren and others are in particular known to undergo physical transformation into undesired crystalline and/or amorphous forms in the course of manufacturing processes including steps wherein the active substance is contacted with moisture and subjected to subsequent drying. One specific example for a manufacturing process where this problem may occur is wet granulation, in particular wet granulation using water or aqueous liquids such as aqueous solutions or dispersions as the granulation liquid. This type of wet granulation is still very common in the manufacturing of solid dosage forms such as tablets and capsules.

For example, it is known that aliskiren hemifumarate is converted to an amorphous form when processed by using aqueous wet granulation using water and/or aqueous binder solutions.

The prior art has attempted to solve the above problems by using alternative granulation methods and liquids. For example, WO 2006/041974. WO 2005/089729 and US 2006/0018960 A1 suggest a process for preparing a formulation based on aliskiren as the active substance using wet granulation of aliskiren using a mixture of organic solvents or a binder solution in organic solvents. Thus, the use of organic solvents is an essential feature of the manufacturing process disclosed in these two prior art documents. The drawback of such a process is that residual solvents and impurities in the product of such solvent-based granulation methods may render the resulting formulation unstable and that drying steps are needed. The prior art discussed above also contains a very general reference to basically any other method of granulation, see. e.g. paragraph [0090] of US 2006/0018960 A1. However, this does not change the essential teaching of the invention of US 2006/0018960 A1 and WO 2005/089729 that using a granulation liquid, i.e. a mixture of organic solvents, is mandatory to achieve a satisfactory result, i.e. to avoid contact with water.

EP-A-1 972 335 and WO-A-2008/116601 relate to a process for the manufacture of a pharmaceutical formulation comprising (a) granulating aliskiren or a pharmaceutical salt thereof alone or in admixture with one or more excipients selected from binders, fillers, disintegrants, wetting agents and/or lubricants at elevated temperature and in the essential absence of solvents i such as those used in the prior art discussed in the preceding paragraph; and (b) optionally formulating the granulate obtained in step (a) into tablets, film-coated tablets, pills, lozenges, sachets, soft or hard gelatine capsules.

However, a need still exists for providing methods for manufacturing formulations containing aliskiren or other moisture-sensitive active substances for in particular effective oral delivery, which methods take account of the demanding characteristics of the active substances, in particular in relation to their processing, and which provide pharmaceutical formulations having an appropriate disintegration time and/or appropriate solubility and/or dissolution profile of the active principle.

### Summary of the Invention

The inventors of the present application have surprisingly found that a certain type of aqueous granulation process can be used for aliskiren and its pharmaceutically acceptable salts, an active substance prone to chemical degradation and/or physical phase transformations (polymorphism) upon contact with water or aqueous liquids, when certain limitations concerning the amount and contacting time of the granulation liquid are observed. In particular, the inventors of the present application have surprisingly succeeded in manufacturing dosage forms having good stability and bioavailability, said dosage forms containing moisture sensitive active substances such as aliskiren and its pharmaceutically acceptable salts, by using a moisture-activated granulation process.

More specifically, in accordance with the present invention there is provided a moisture-activated granulation process for the manufacture of a pharmaceutical dosage form containing at least one moisture-sensitive active substance selected from the group consisting of aliskiren and its pharmaceutically acceptable salts, esters or cocrystals, said process comprising:
a) mixing the active substance(s) with one or more dry excipients in solid form, said excipient being selected in particular from the group consisting of diluents, surfactants, binders, fillers, lubricants, disintegrators, granulation aids, buffering/alkalizing agents and antioxidants;
b) contacting the mixture with a granulation liquid containing water and optionally one or more excipients to form a granulate, said excipient being selected in particular from the group consisting of binders, buffering/alkalizing agents, surfactants and antioxidants, to form a granulate wherein the ratio of the total amount of water to the total amount of all the solid ingredients including the active substance(s) and all intragranular excipients is less than 30:100, based on weight; and
c) further processing the mixture including the addition of dry excipients to obtain the pharmaceutical dosage form, said excipients being selected in particular from the group consisting of diluents, disintegrants, glidants and lubricants.

The present invention also provides a pharmaceutical dosage form containing a moisture-sensitive active substance said formulation being obtainable by a process as defined above.

The present invention also provides a pharmaceutical dosage form containing a moisture-sensitive active substance as defined herein for use in the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache, chronic heart failure, erectile dysfunction, and female sexual dysfunction such as female arousal disorder.

Further preferred embodiments of the present invention are disclosed in the dependent claims attached to this description.

### Brief Description of the Figures

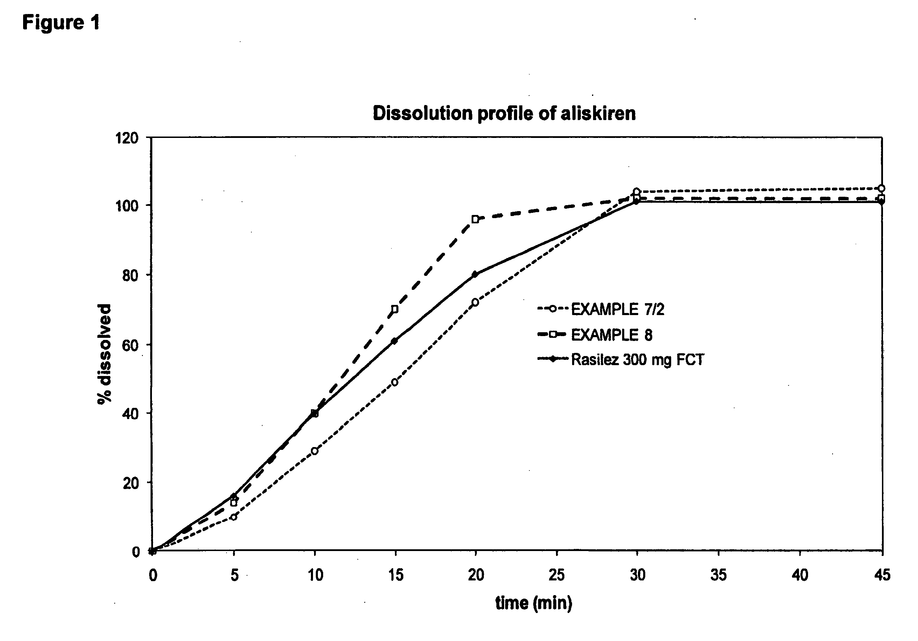

Figure 1 shows comparative *in-vitro* dissolution profiles of aliskiren in the form of the hemifumarate salt from tablets prepared according to Example 7/2 and Example 8 as well as Rasilez 300 mg FCT (S0069) in 1000 mL 0.1M HC1 at 37°C in an Apparatus 1 - basket (Ph.Eur. or USP, 100 rpm).

### Detailed Description of the Invention

In accordance with the present invention, the term "dry" means that the water content of the excipients which are mixed with the one or more active ingredient in step (a) is less than 2 wt-%, preferably less than 1.5 wt-%, based on the total weight of the excipient mixed with the active ingredient and determined by the loss on drying method according to Ph. Eur. Chapter 2.2.32 (determined on 1.000g by drying in an oven at 105°C for 3 hours). Of course, the active substance will also be used in the starting mixture of step (a) as a dry component.

Furthermore, in the description of the moisture-activated granulation process for the manufacture of the pharmaceutical dosage forms of the present invention, reference is made to intragranular excipients. This term means those excipients which are used during the preparation of the granulate, i.e. during steps (a) and (b). Excipients which are added during the further processing of the granulate into dosage forms such as tablets may be referred to as extragranular excipients.

Preferably, the ratio of the total amount of water to the total amount of all the solid ingredients including the active substance(s) and all intragranular excipients is less than 20:100, in particular less than 10:100, based on weight.

In particular, the granulation liquid of the present invention consists of water, purified in accordance with pharmaceutical requirements.

In case excipients such as binders, buffering/alkalizing agents, surfactants or antioxidants are dissolved or dispersed in the water of the granulation liquid, they are taken into account in the calculation of the above ratios as solid, dry excipients.

In general, the excipients and the active ingredient (s) which are mixed in step (a) are present in the form of finely divided solid particles, e.g. as powders. Usually, the particles have an average particle size in the range of micrometers, e.g. 0.1 and up to 500 µm, preferably 0.5 to 450 µm. Preferably, the average particle size of the active ingredients is in the range from 0.5 to 450 µm, preferably 1 to 400 µm. In case particles of active substance have a tendency of forming agglomerates, it is preferred that the average particle size of primary particles of active substances used in the preparation of the solid composition according to present invention is in the range of from 0.5 to 150 µm. D90 of active ingredient is below 900 µm, preferably below 800 µm. Average particle size and D90 are preferably determined by laser diffraction using equipment such as Malvern instruments. Preferably, the dry components are mixed in the form of powders.

In the following description, when referring to "aliskiren" this includes the pharmaceutically acceptable salts thereof such as in particular aliskiren hemifumarate.

The pharmaceutical formulations of the invention are preferably provided in a solid unit dosage form, each dosage containing about 1-600 mg of aliskiren in its free form. In case the hemifumarate salt of aliskiren is used, a therapeutic dose of 75 mg, 150 mg, 300 mg and 600 mg of free aliskiren corresponds to 82.88 mg, 165.75 mg, 331.50 mg and 663.00 mg of aliskiren hemifumarate, respectively.

Methods for obtaining aliskiren and its pharmaceutically acceptable salts are known in the art. For example, EP 678 503 describes the hydrochloride salt (Example 137) and the hemifumarate salt (Example 83) as specific salts of aliskiren.

Further multistage syntheses of substituted octanoyl amides and concrete examples for preparation of aliskiren hemifumarate are for example described in EP 1200390, EP 1296935, EP 1303478 and EP 2062874. Any form of aliskiren or its salts as obtainable by said methods can be used in the present invention, in particular as described by Examples G1, J1 or K1 of:
EP 1200390 = WO 2001/009083 (Example G1)
EP 1296935 = WO 2002/002508 (Example J1)
EP 1303478 = WO 2002/008172 (Example K1)
and by Example 1 of EP 2062874.

Further specific crystalline forms of aliskiren hemifumarate are described in WO 2008/061622. In particular, reference is made to the crystalline forms described as Modification A, Modification B, Solvate Form S_{A} and S_{B}, Types I, II, III, IV, V and VI, as described on pages 4 to 6 and Figures 1 to 11 as well as on pages 7, bottom paragraph to page 10, penultimate paragraph of this International patent application. Reference is also made to the definition of the crystal forms in claims 1 to 37 of WO 2008/061622.

Furthermore, the solid forms of aliskiren, aliskiren hemifumarate and aliskiren monofumarate as described in WO 2009/149344, WO 2009/064479 and WO 2009/143423, respectively, can be used in the process of the present invention.

Surprisingly we have found out that the crystalline form of active ingredient does not change during a manufacturing procedure of a solid composition according to present invention.

In addition to the crystalline hemifumarate salts of aliskiren, hydrogen sulphate, nitrate, and orotate are known from WO 2007/107317, WO 2007/098503 and WO 2008/055669, respectively, and can be used in the process of the present invention.

The term "unit dosage form" refers to physically discrete units suitable as unitary dosages for humans and other mammals, each containing a predetermined quantity of active ingredient calculated to produce the desired therapeutic effect, in association with a suitable pharmaceutical excipient.

In the process of the present invention, the ratio of the total amount of water to the total amount of all the solid ingredients including the active substance(s) and all intragranular excipient is less than 30: 100, preferably less than 20:100, in particular less than 10:100, based on weight. In particular, the amount and contacting time of the mixture of the active ingredient(s) and excipients are selected such that a measurable chemical degradation and/or undesired physical phase transition does not occur, although a granulation liquid is used which is known for the respective active substance to have in principle the potential of causing such undesired changes. The concentration of dissolved or dispersed solid components in the granulation liquid, if present, is less than 25 weight%, preferably less than 15 weight%.

The following sections describe the details of the excipients used in the present invention. In general, excipients are used which are required to prepare a stable pharmaceutical dosage form such as in particular a tablet.

More specifically, in accordance with the present invention, diluents can be selected from soluble saccharides such as mannitol, xylitol, powdered cellulose, microcrystalline cellulose, starch and its derivatives, alkaline earth metal phosphates such as calcium hydrogen phosphate in hydrated or anhydrous form.

In order to achieve good processibility during compression, the particle size of the diluents is important. In case of diluents used intragranularly, the average particle size determined by laser diffraction method such as by Malvern instrument can be in the range from 10 to 150 µm, preferably 25 to 120 µm. The average particle size of diluents used in the extragranular phase can be in the range from 50 to 400 µm preferably 75 to 350 µm. Diluents especially designed for direct compression having physically modified particles obtained by state of the art processes such as spray drying and/or granulation are especially preferred for use in the extragranular phase. Coprocessed diluents having improved compressibility and flowability such as silicified microcrystalline cellulose alone or coprocessed with additional excipients such as disintegrants and lubricants (e.g. different Prosolv types obtainable from Penwest), a combination of microcrystalline cellulose and lactose such as MicrocelLac obtainable from BASF) can be used.

Surfactants can in general be selected from nonionic or ionic surfactants having an HLB value of more than 8, preferably of more than 10. Nonionic surfactants can in particular be selected from polyoxyethylated glycol monoethers, cetomacrogol, sorbitan esters (Spans) and polysorbates (Tweens), polyoxyethylenepolyoxypropylene copolymers such as poloxameres, sugar esters with fatty acids with 10 to 22 C atoms. Ionic surfactants can in particular be selected from the group of anionic surfactants such as organic sulphonates (RSO₃⁻), sulphates (ROSO₃⁻) e.g. sodium lauryl sulphate CH₃(CH₂)₁₁SO₄⁻Na⁺, potassium laurate CH₃(CH₂)₁₀COO⁻K⁺ or cationic surfactants selected from the group consisting of organic quaternary ammonium halides, R₄N⁺Cl⁻, cetrimide, a mixture consisting of tetradecyl (about 68%), dodecyl (about 22%), and hexadecyltrimethylammonium bromides (about 7%), as well as benzalkonium chloride, a mixture of alkylbenzyldimethylammonium chlorides of the general formula [C₆H₅CH₂N⁺(CH₃)₂R]Cl⁻, where R represents a mixture of alkyls from C₈H₁₇ to C₁₈H₃₇ or ampholytic surfactants selected from sulfobetaines RN⁺(CH₃)₂CH₂CH₂SO₃⁻), or betains N-Dodecyl-N,N-Dimethylbetaine, C₁₂H₂₅N⁺(CH₃)₂CH₂COO⁻ .

Binders can be selected from povidone with a K value of from 7 to 100, copovidone, polyvinyl alcohol, block copolymer of ethylene oxide and vinyl alcohol sold under trade name Kollicoat IR, microcrystalline cellulose, water soluble types of cellulose ethers such as hydroxyethylcellulose, hydroxypropylcellulose, hydroxypropylmethylcellulose, starch, pregelatinised starch, microcrystalline cellulose. Povidone with a K value of from 7 to 100 such as Povidone K25 or K30 is preferred.

Lubricants for use in the present invention are in general selected from metal stearates comprising magnesium, calcium, sodium, aluminium or zinc stearate, talc, hydrogenated castor oil, sodium stearyl fumarate, partial fatty esters of glycerol such as glycerol monostearate, fatty acids such as stearic acid. Preferably, talc or magnesium stearate are used.

Disintegrants are in general selected from the group consisting of crospovidone (e.g. Kollidon Cl of type A or B as decribed by Volker Bühler in Kollidon®, Polyvinylpyrollidone excipients for the pharmaceutical industry; published by BASF SE in March 2008, pp.: 143-203 or Polyplasdone sold by company ISP such as Polyplasdone INF-10 (average particle size in the range 5-10 µm), Polyplasdone XL average particle size in the range 100-130 µm)or Polyplasdone XL-10 average particle size in the range 30-50 µm), starch, pregelatinised starch, sodium starch glycollate, microcrystalline cellulose, carboxymethylcellulose sodium (CMC-Na), also called croscarmellose sodium, or calcium (CMC-Ca), crosslinked CMC-Na, polacrilin potassium, low-substituted hydroxypropylcellulose or mixtures thereof. A particularly preferred disintegrant is crospovidone.

Granulation aids, which are used to prevent stickiness of the composition during granulation and subsequent compression, can be selected from substances having surface area of at least 0,25 m²/g, preferably 0,5 m²/g, more preferably 1 m²/g and even more preferably of more than 50 m²/g. Granulation aids can be hydrophilic such as colloidal anhydrous silica sold under trade name Aerosil 200, bentonite, talc, zeolite, porous silicone dioxide sold under trade name Sylysia 350 or magnesium aluminometasilicate sold under trade name Neusilin, and types such as UFL2 and US2. Hydrophobic excipients used as granulation aids can be selected from, but are not limited to, fatty acids having 10 to 24 carbon atom such as stearic acid, metal salts of fatty acid having 10 to 24 carbon atom such as magnesium, calcium, sodium, aluminium or zinc salts of stearic and/or palmitic acid, hydrogenated castor or vegetable oil and hydrophobic colloidal anhydrous silica sold under trade name Aerosil R972. Most preferably, hydrophobic or hydrophilic colloidal silica sold under trade name Aerosil R972 or Aerosil 200D are used.

Buffering/alkalizing agents can be selected from the group of pH modifiers such as moderately acidic or alkaline reacting compounds. When incorporated into the solid dosage form containing aliskiren in the form of salts, they buffer the pH of the solid composition in the range of 6 to 8, preferably 6.5 to 7.5. The pH of the solid composition is measured by dispersing a crushed tablet in 200 ml of purified water.

Specifically, the buffering/alkalizing agents can in general be selected from substances such as sodium or potassium hydroxide, ammonia solution, mono or dibasic alkali and alkaline earth metal phosphates such as disodium hydrogen phosphate in anhydrous or hydrated state, calcium hydrogen phosphate in anhydrous or hydrated state, alkali and alkaline earth metal hydrogen carbonates, earth alkali carbonates and hydroxy carbonates such as magnesium carbonate; heavy alkaline earth oxides such as magnesium oxide, alkali and alkaline earth metal salts of polycarboxylic acids such as citric acid or mixtures thereof. The buffering/alkalizing agents can be added partly of fully either in dissolved form in the granulation liquid or in a solid form such as in powder form to the dry components of the granulate.

Antioxidants can be selected from butylhydroxyanizole (BHA), butylhydroxytoluene (BHT), ascorbic acid, ascorbic acid metal salts, α-tocopherole and its salts, pharmaceutically acceptable substances having kinone moiety in the molecule such as gallic acid, caffeic acid, quercetin, rutin or combination thereof, and the like.

In a preferred embodiment of the process of the present invention, the active ingredient such as aliskiren is mixed with a combination of a granulation aid based on highly dispersed silica such as colloidal anhydrous silica or colloidal hydrophobic silica, a binder based on a povidone such as povidone with a K value of from 7 to 100, and optionally other excipients such as diluents (e.g. manitol, powdered cellulose, xylitol), glidants/lubricants such as talc and optionally part or the whole amount of buffering/alkalizing agents selected from the group of sodium or potassium hydroxide, ammonia solution, mono or dibasic alkali and alkaline earth metal phosphates. All components are present as powders (step (a) of the process of the invention).

After sufficient mixing, the components are contacted with a granulation liquid which contains water, in a ratio as defined hereinabove, and optionally a buffering/alkalizing agents selected from the group of sodium or potassium hydroxide, ammonia solution, mono or dibasic alkali and alkaline earth metal phosphates, to form a granulate. Optionally, the granulation liquid further contains a part or all of the binder used in the pharmaceutical formulation.

The terms "contact time" or "contacting time" as used herein describe the time period during which the granulation liquid is contacted with, e.g. sprayed onto the powder mixture excluding drying time.

On the basis of the information provided herein, it will be understood by the person skilled in the art that the contacting time may vary depending on the scale of the process and the apparatus and detailed conditions used.

According to the present invention, shorter contact times of granulation liquid with solid mixture than known from the prior art are required. The contact time may typically be in the range from 30 seconds to 5 minutes.

After granulating the components by using a suitable device such as preferably a fluid-bed or high-shear granulator, further excipients can and preferably are added. Such excipients are preferably selected from the group of diluents, especially low moisture grades of microcrystalline cellulose, glidants such as highly dispersed silica such as colloidal anhydrous silica, disintegrants such as crospovidone, and lubricants such as magnesium stearate and talc.

If necessary, the further processing of the mixture after the granulation step can include a drying step and a sieving step.

Examples for suitable preferred granulation aids which can be used in steps (a) and (b) of the process of the present invention are silica materials such as colloidal anhydrous silica having a hydrophilic surface, e.g. Aerosil 200D, or a hydrophobic surface, e.g. Aerosil R972, and a specific surface area determined by B.E.T. method (adsorption of nitrogen) of more than 10m²/g, preferably of more than 50m²/g and even more preferably in the range of 100 to 600m²/g. Preferred dilluents are cellulose materials such as microcrystalline cellulose, especially low moisture grades. Suitable lubricants are metal stearates such as alkaline earth metal stearates, for example, magnesium stearate.

In a particularly preferred embodiment of the process of the present invention, aliskiren hemifumarate is used as the active ingredient and is processed as follows.

The active ingredient is preferably mixed with a combination of an excipient from the group of granulation aids, in particular Aerosil R972, a povidone binder, in particular povidone K25 or K30, a diluent such as mannitol or powdered cellulose, glidants/lubricants such as talc and optionally part or the whole amount of buffering/alkalizing agents selected from the group of sodium or potassium hydroxide, mono or dibasic alkali and alkaline earth metal phosphates. All components are present as powders (step (a) of the process of the invention).

After sufficient mixing, the components are contacted with the granulation liquid which contains water, in a ratio as defined hereinabove, and optionally a buffering/alkalizing agents selected from the group of sodium or potassium hydroxide, mono or dibasic alkali metal phosphates, in particular dibasic or tribasic sodium phosphate or calcium hydrogen phosphate dihydrate.

In this context, it is noted that in order to improve the chemical stability of the drug in the final solid composition, it is advantageous to control the drying process of granulate. As discussed above, aliskiren and its pharmaceutically acceptable salt undergo chemical degradation in presence of moisture especially in solid compositions containing them. In the present case, the moisture content in the granulate can be controlled to improve the stability of aliskiren and its pharmaceutically acceptable salt in the granulate and in the final solid composition manufactured by mixing the granulate with other excipients and compressing the mixture into tablets. The stability of aliskiren and its pharmaceutically acceptable salt is generally evaluated through the content of individual and total (sum of) impurities and degradation products present in the solid composition upon storage. The content of individual and total impurities is limited by general regulatory requirements. The data obtained by the present inventors show that the extent of chemical degradation of aliskiren and its pharmaceutically acceptable salt in a solid composition such as tablet is also dependent on the moisture content of the granulate prepared by the process of the present invention. On the basis of the results of stress stability tests as shown herein, which tests are indicative of the stability of solid compositions on storage, it is preferred that the residual moisture in granulate is kept below about 2 weight%, preferably below about 1.7 weight% and most preferably below about 1.5 weight%, or in case of analyzing tablet cores or film coated tablets, the residual moisture is kept below about 5 weight%, preferably 4.5 weight% and most preferably 3.8 weight% as determined by a halogen dryer which is used as moisture analyzer (e.g. Mettler HR 73; approx 3 - 4 g of granulate or crushed tablets. In case of analyzing tablets (cores or film coated tablets), they are crushed (using mortar and pestle) and transferred to the moisture analyzer. The moisture content is then determined at a drying temperature of 105°C and a drying time of 5 minutes.

After granulating the components using a fluid-bed or high-shear granulator, extragranular diluents such as microcrystalline cellulose, especially low moisture grades and optionally a portion of colloidal anhydrous silica having a hydrophilic or hydrophobic surface, in particular (Aerosil 200D, or coprocessed silified microcrystalline cellulose, are added. If necessary, i.e. optionally, the mixture is further dried and sieved.

Afterwards, an additional portion of microcrystalline cellulose, especially low moisture grades and optionally colloidal anhydrous silica, disintegrant (especially crospovidone) and finally a lubricant, e.g. a metal stearate, in particular magnesium stearate, are added.

The pharmaceutical formulation of the present invention can be further processed to oral dosage forms such as tablets or capsules, which optionally can be further coated with a functional film coating. Thereby, the dosage forms are provided with the required physical or biopharmaceutical properties such as resistance against dissolution in acidic media (gastro resistant coating), decreased permeability to moisture and other gases such as oxygen, taste or colour masking and/or smoothening of the surface for easier swallowing in case of tablets.

For example, a gastro resistant coating can be based on polymers insoluble at pH below 7.5, preferably below 6.5, most preferably below 6. Further excipients for use in such coatings are selected from plasticizers, antitacking agents, pigments and colorants, and glidants.

Film coating of tablet cores based on polymers such as polyvinyl alcohol such as Opadry AMB and/or Kollicoat Protect or methacrylic acid derivatives such as Eudragit EPO having decreased permeability for moisture and other gases can be used. Film coating can optionally include other pharmaceutically acceptable inactive ingredient selected from plasticizers such as polyethylene glycol, propylene glycol, antitacking agents such as talc, glycerol monostearate, magnesium stearate, stearic acid, pigments such as metal oxides like iron oxides, titanium dioxide and colorants.

Coated tablets of aliskiren hemifumarate obtained according to the present invention tested with the dissolution method described in Example 12 show dissolution results of more than 80% aliskiren hemifumarate dissolved after 30 minutes.

In a specific embodiment of present invention, the active pharmaceutical ingredient or its salts cocrystal or ester such as aliskerene hemifumarate can be combined with at least one further active ingredient selected from diuretics, antihypertensives, lipid regulators and antidiabetics in the form of free acids, free bases, salts or esters. Further active ingredient(s) can be incoporated into aliskiren solid composition either:
1. ntragranularly together with aliskiren, wherein the obtained granulate is processed into tablets or capsules as described above; or
2.extragranularly, wherein
   a. the further active ingredient can be formulated into a separate granulate by methods known from the state of the art. In such a case, a first granulate containing aliskiren and obtained in accordance with the present invention is mixed with a second granulate containing at least one further active substance and optionally other excipients selected from diluents, binders, disintegrants, glidants and/or lubricants, and is compressed into tablets or filled into capsules; or
   b. the further active ingredient can be mixed with the granulate containing aliskiren alone or with a granulate obtained as described under point 1 or 2.a above in powder form and compressed into tablets or filled into capsules. Further excipients selected from diluents, binders disintegrants, glidants and/or lubricants can be admixed to the obtained mixture before compression.

In a further embodiment, the present invention relates to combinations of aliskiren with 2 or more further active ingredients such as:
Antihypertensives selected from but not limited to the following pharmacological subgroups: angiotensin converting enzyme (ACE) inhibitors, angiotensin receptor blockers (ARBs), AT₁-receptor antagonists, calcium-channel blockers (CCBs) and anti-adrenergics.

For example, ACE inhibitors may be selected from the group consisting of captopril, enalapril, lisinopril, trandolapril, cilazapril, ramipril, fosinopril, perindopril or a pharmaceutically acceptable salt thereof.

AT₁-receptor antagonist for use in the combined formulation may be selected from the group consisting of candesartan, irbesartan, losartan, olmesartan, telmisartan, valsartan or pharmaceutically acceptable salts thereof.

The calcium channel blocker may be selected from the group consisting of amlodipine, diltiazem, felodipine, nifedipine, nitrendipine and verapamil and salts thereof.

From the group of β-adrenergic blockers compounds like acebutol, atenolol betaxolol, bisoprolol, metoprolol, and pharmaceutically acceptable salts thereof can be selected. As mixed α-and β-adrenergic blockers, carvedilol may be included in the formulation.

Lipid regulators for combination with aliskiren in the formulation of the present invention can be selected from 3-hydroxy-3methylglutaryl coenzyme A (HMG CoA) reductase inhibitors such as lovastatin, simvastatin, pravastatin, atorvastatin, fluvastatin, cerivastatin, rosuvastatin, and pharmaceutically acceptable salts thereof.

The antidiabetic agent may be selected from the group of sulfonyl urea, meglitinides (such as nateglinide, repaglinide) and pharmaceutically acceptable salts thereof, thiazolidinediones (such as pioglitazone, rosiglitazone) and pharmaceutically acceptable salts thereof, alpha glucosidase inhibitors, incretin mimetics, or biguanides such as metformin or the like and the pharmaceutically acceptable salts thereof.

The pharmaceutical formulation of the present invention can be packaged in accordance with procedures and materials known from the state of the art. For example, as packaging material blisters, glass bottles, containers made of polymeric materials with suitable closure systems may be used. Packaging can be performed under normal atmosphere or optionally under an atmosphere having a reduced oxygen concentration, preferably in an inert atmosphere or under reduced relative humidity such as 40% RH, preferably 35% RH and most preferably below 30% RH. Reduced oxygen concentration means that the concentration of oxygen in the gas surrounding the solid composition in the primary packaging is below 18 vol/vol%, preferably below 10 vol/vol% and most preferably below 5vol/vol%.

Any pharmaceutical composition may be prepared and stored in a packaging material usually chosen by those of ordinary skill in the art of maintaining stability of active drugs. Decreased moisture permeability of primary packaging material is preferred in order to diminish moisture sorption by aliskiren containing dosage forms to avoid any changes in drug stability. Low moisture permeable primary packaging materials such as aluminium or polychloro-3-fluoroethylene homopolymer/PVC laminate can be used with the thickness in the range 10 to 40 µm in case of Al/Al blisters and 10 to 110 µm in case of Al-polychloro-3-fluoroethylene homopolymer/PVC laminate blisters. Optionally, dosage forms containing aliskiren or its pharmaceutically acceptable salts can be packed into primary packaging with desiccant. Desiccant can be placed inside the packaging unit together with aliskiren dosage units such as tablets and/or in the closure system or can be incorporated into the walls of the primary packaging unit.

According to a preferred embodiment, contact between the pharmaceutical composition and environmental oxygen may be reduced or suppressed by either packaging the pharmaceutical composition under reduced pressure, packaging in an inert gas atmosphere, using a coating affording protection and stability of the pharmaceutical composition to environmental influences, or by using a packaging wherein the contact between the pharmaceutical composition and oxygen is reduced by the means of oxygen absorbers.

An atmosphere with reduced oxygen content or reduced oxygen partial pressure may be obtained by the use of an atmosphere of reduced pressure, e.g. by creating a partial vacuum by means of a suitable pump or by partial freezing or liquefying the atmosphere, by the use of an inert gas atmosphere, wherein as an inert gas nitrogen or argon may be used, or by the use of absorbents. Suitable absorbents may be selected from the group of commercially available absorbents such as humidityactivated oxygen absorbers, ultraviolet-radiation-activated absorbers, radiation-activated absorbers, microwaves-radiation-activated absorbers, absorbers activated by a combination of activation processes or absorbers without necessity of activation. Examples of commercially available absorbers are Ageless™ (Mitsubishi Gas Chemical), ATCO (Standa Industry), FreshPax™ (Multisorb Technologies), O-Buster™ (Hsiao Sung Non-Oxygen Chemical Co), Biotika Oxygen Absorber (Biotika) and the like.

The invention also provides a stabilized package of aliskiren which is provided with a space for trapping and disposal of free oxygen. Moreover, if the active compounds of the present composition are exhibited to a reduced oxygen partial pressure, the formulation is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Al-polychloro-3-fluoroethylene homopolymer/PVC laminate blister.

Final packaging can optionally contain dessicant which can be used in blisters made of aluminium foil, incorporated into closure system of glass and/or polymeric containers or added directly into containers.

The following examples further illustrate the present invention.

### Example 1

| **Component** | **Amount [mg/tablet]** |
|---|---|
| Aliskiren hemifumarate (corresponding to 300mg of aliskiren) | 331,50 |
| Colloidal anhydrous hydrophobic silica (Aerosil R 972) | 5,00 |
| Talc | 15,00 |
| Ca hydrogen phosphate, dihydrate | 88,50 |
| Povidone K25 | 40,00 |
| Dibasic sodium phosphate (Na2HPO4) | 4,00 |
| Water | q.s. |
| **Granulate (before drying)** | *484,00* |
| Microcrystalline cellulose (Avicel PH 200 LM) | 219,00 |
| Colloidal anhydrous silica (Aerosil 200 D) | 5,00 |
| Magnesium stearate | 12,00 |
| **Tablet core** | *720,00* |
| Film coating (Opadry PINK OY-S-24900) | 20 |

### Manufacturing procedure:

The active ingredient was mixed with with colloidal anhydrous hydrophobic silica (Aerosil R972), Ca hydrogen phosphate dihydrate, talc and povidone in powder form. Then, the granulation liquid consisting of purified water and disodium hydrogenphosphate (dibasic sodium phosphate) was sprayed onto the powder mixture and granulating/kneading was performed for totally 100 seconds using a Mi-Pro high-shear granulator. Next, colloidal anhydrous silica (Aerosil 200 D), microcrystalline cellulose, (Avicel PH 200LM) were added and mixed with the other components for 60 seconds. After drying and sieving, magnesium stearate was added as lubricant. Finally, the mixture was compacted into tablets and provided with a film-coating.

### Example 2

| **Component** | **Amount [mg/tablet]** |
|---|---|
| Aliskiren hemifumarate (corresponding to 150mg of aliskiren) | 165,75 |
| Colloidal anhydrous hydrophobic silica (Aerosil R 972) | 3,50 |
| Talc | 11,00 |
| Mannitol | 45,85 |
| Povidone K30 | 25,00 |
| Sodium hydroxide | 0,40 |
| Water | 20,00 |
| **Granulate (before drying)** | *251,50* |
| Microcrystalline cellulose (Avicel PH 200 LM) | 53,00 |
| Microcrystalline cellulose (Avicel PH 200 LM) | 43,00 |
| Crospovidone | 5,00 |
| Colloidal anhydrous silica (Aerosil 200 D) | 2,50 |
| Castor oil, hydrogenated | 15,00 |
| **Tablet core** | *370,00* |
| Film coating (Opadry PINK OY-S-24900) | 10 |

The active ingredient was mixed with with colloidal anhydrous hydrophobic silica (Aerosil R972), talc, mannitol, povidone and sodium hydroxide in powder form. Then, the granulation liquid consisting of purified water was sprayed onto the powder mixture. Next, microcrystalline cellulose, (Avicel PH 200LM) was added and granulating/kneading was performed for 1 minute using a Mi-Pro high-shear granulator. After drying and sieving, another portion of microcrystalline cellulose, (Avicel PH 200LM), crospovidone and colloidal anhydrous silica (Aerosil 200 D) were added and mixed with the other components. Afterwards castor oil hydrogenated was added as lubricant. Finally, the mixture was compacted into tablets.

### Example 3

| **Component** | **Amount [mg/tablet]** |
|---|---|
| Aliskiren hemifumarate (corresponding to 150mg of aliskiren) | 165,75 |
| Colloidal anhydrous hydrophobic silica (Aerosil R 972) | 3,50 |
| Talc | 11,00 |
| Xylitol | 45,85 |
| Povidone K30 | 25,00 |
| Sodium hydroxide | 0,40 |
| Water | 25,00 |
| **Granulate (before drying)** | *251,50* |
| Microcrystalline cellulose (Avicel PH 200 LM) | 53,00 |
| Microcrystalline cellulose (Avicel PH 200 LM) | 43,00 |
| Crospovidone | 5,00 |
| Colloidal anhydrous silica (Aerosil 200 D) | 2,50 |
| Castor oil, hydrogenated | 15,00 |
| **Tablet core** | *370*,*00* |

The active ingredient was mixed with with colloidal anhydrous hydrophobic silica (Aerosil R972), talc, xylitol, povidone and sodium hydroxide in powder form. Then, the granulation liquid consisting of purified water was sprayed onto the powder mixture. Next, microcrystalline cellulose, (Avicel PH 200LM) was added and granulating/kneading was performed for 1 minute using a Mi-Pro high-shear granulator. After drying and sieving, another portion of microcrystalline cellulose, (Avicel PH 200LM), crospovidone and colloidal anhydrous silica (Aerosil 200 D) were added and mixed with the other components. Afterwards castor oil hydrogenated was added as lubricant. Finally, the mixture was compacted into tablets.

### Example 4

| **Component** | **Amount [mg/tablet]** |
|---|---|
| Aliskiren hemifumarate (corresponding to 150mg of aliskiren) | 165,75 |
| Colloidal anhydrous hydrophobic silica (Aerosil R 972) | 3,50 |
| Talc | 7,50 |
| Powdered cellulose | 47,35 |
| Povidone K30 | 27,00 |
| Sodium hydroxide | 0,40 |
| Water | 30,00 |
| **Granulate (before drying)** | *251,50* |
| Microcrystalline cellulose (Avicel PH 200 LM) | 58,30 |
| Microcrystalline cellulose (Avicel PH 200 LM) | 46,70 |
| Crospovidone | 5,00 |
| Colloidal anhydrous silica (Aerosil 200 D) | 2,50 |
| Magnesium stearate | 6,00 |
| **Tablet core** | *370,00* |

The active ingredient was mixed with with colloidal anhydrous hydrophobic silica (Aerosil R972), talc, powdered cellulose, povidone and sodium hydroxide in powder form. Then, the granulation liquid consisting of purified water was sprayed onto the powder mixture. Next, microcrystalline cellulose, (Avicel PH 200LM) was added and granulating/kneading was performed. After drying and sieving, another portion of microcrystalline cellulose, (Avicel PH 200LM), crospovidone and colloidal anhydrous silica (Aerosil 200 D) were added and mixed with the other components. Afterwards magnesium stearate was added as lubricant. Finally, the mixture was compacted into tablets.

### Example 5

| **Component** | **Amount [mg/tablet]** |
|---|---|
| Aliskiren hemifumarate (corresponding to 150mg of aliskiren) | 165,75 |
| Colloidal anhydrous hydrophobic silica (Aerosil R 972) | 3,50 |
| Talc | 7,50 |
| Powdered cellulose | 47,35 |
| Povidone K30 | 27,00 |
| Sodium hydroxide | 0,40 |
| Water | 40,00 |
| **Granulate (before drying)** | *251,50* |
| Microcrystalline cellulose (Avicel PH 200 LM) | 105,00 |
| Crospovidone | 5,00 |
| Colloidal anhydrous silica (Aerosil 200 D) | 2,50 |
| Magnesium stearate | 6,00 |
| **Tablet core** | *370,00* |

The active ingredient was mixed with with colloidal anhydrous hydrophobic silica (Aerosil R972), talc, powdered cellulose and povidone in powder form. Then, the granulation liquid consisting of purified water and sodium hydroxide was sprayed onto the powder mixture for 4 minutes using a GPCG-1 fluid-bed granulator with top position of spray nozzle.

After drying and sieving microcrystalline cellulose, (Avicel PH 200LM), crospovidone and colloidal anhydrous silica (Aerosil 200 D) were added and mixed with the other components. Afterwards magnesium stearate was added as lubricant. Finally, the mixture was compressed into tablets.

### Example 6

| **Component** | **Amount [mg/tablet]** |
|---|---|
| Aliskiren hemifumarate (corresponding to 150mg of aliskiren) | 165,75 |
| Colloidal anhydrous hydrophobic silica (Aerosil R 972) | 3,50 |
| Talc | 7,50 |
| Powdered cellulose | 47,35 |
| Povidone K30 | 27,00 |
| Sodium hydroxide | 0,40 |
| Water | 30,00 |
| **Granulate (before drying)** | *251,50* |
| Microcrystalline cellulose (Avicel PH 200 LM) | 105,00 |
| Crospovidone | 5,00 |
| Colloidal anhydrous silica (Aerosil 200 D) | 2,50 |
| Magnesium stearate | 6,00 |
| **Tablet core** | *370,00* |

The active ingredient was mixed with with colloidal anhydrous hydrophobic silica (Aerosil R972), talc, powdered cellulose and povidone in powder form. Then, the granulation liquid consisting of purified water and sodium hydroxide was sprayed onto the powder mixture for 3 minutes using a GPCG-1 fluid-bed granulator with top position of spray nozzle.

After drying and sieving, microcrystalline cellulose, (Avicel PH 200LM), crospovidone and colloidal anhydrous silica (Aerosil 200 D) were added and mixed with the other components. Afterwards magnesium stearate was added as lubricant. Finally, the mixture was compressed into tablets.

### Example 7

| **Component** | **Amount [mg/tablet]** |
|---|---|
| Aliskiren hemifumarate (corresponding to 300mg of aliskiren) | 331,50 |
| Colloidal anhydrous hydrophobic silica (Aerosil R 972) | 7,00 |
| Talc | 40,00 |
| Powdered cellulose | 69,70 |
| Povidone K30 | 54,00 |
| Sodium hydroxide | 0,80 |
| Water | 85,00 |
| **Granulate (before drying)** | *503,00* |
| Microcrystalline cellulose (Avicel PH 200 LM) | 172,00 |
| Crospovidone | 40,00 |
| Colloidal anhydrous silica (Aerosil 200 D) | 5,00 |
| Magnesium stearate | 20,00 |
| **Tablet core** | *740*,*00* |

The active ingredient was mixed with colloidal anhydrous hydrophobic silica (Aerosil R972), talc, powdered cellulose and povidone in powder form. Then, the granulation liquid consisting of purified water and sodium hydroxide was sprayed onto the powder mixture for 4 minutes using a GPCG-1 fluid-bed granulator with top position of spray nozzle.

After drying and sieving, microcrystalline cellulose, (Avicel PH 200LM), crospovidone and colloidal anhydrous silica (Aerosil 200 D) were added and mixed with the other components. Afterwards magnesium stearate was added as lubricant. Finally, the mixture was compressed into tablets. Preferably, tablets are film coated using a standard water based film coating using HPMC.

As can be seen from Figure 1, the dissolution profile of tablets prepared according to above composition and manufacturing procedure is comparable to that of the reference product.

### Example 8

| **Component** | **Amount [mg/tablet]** |
|---|---|
| Aliskiren hemifumarate (corresponding to 300mg of aliskiren)* | 331,50 |
| Colloidal anhydrous hydrophobic silica (Aerosil R 972) | 7,00 |
| Talc | 40,00 |
| Powdered cellulose (Arbocel M80) | 69,70 |
| Povidone K30 | 54,00 |
| Sodium hydroxide | 0,80 |
| Water | 62,50 |
| **Granulate (before drying)** | *503,00* |
| Microcrystalline cellulose (Avicel PH 200 LM) | 169,50 |
| Crospovidone (Kollidon CL) | 42,50 |
| Colloidal anhydrous silica (Aerosil 200 D) | 5,00 |
| Magnesium stearate | 20,00 |
| **Tablet core** | *740,00* |

| | |
|---|---|
| * aliskiren hemifumarate according to EP 2062874, Example 1 (X-ray, FT-IR, DSC) Volume mean diameter 310 µm. Method: Pharm. Eur. 2.9.31, Version 6.6., Malvern Mastersizer. | |

The active ingredient was mixed with with colloidal anhydrous hydrophobic silica (Aerosil R972), talc, powdered cellulose and povidone in powder form. Then, the granulation liquid consisting of purified water and sodium hydroxide was sprayed onto the powder mixture for 3 minutes using a GPCG-1 fluid-bed granulator with top position of spray nozzle.

After drying and sieving, microcrystalline cellulose, (Avicel PH 200LM), crospovidone and colloidal anhydrous silica (Aerosil 200 D) were added and mixed with the other components. Afterwards magnesium stearate was added as lubricant. Finally, the mixture was compressed into tablets. Preferably, tablets are film coated using a standard water based film coating using HPMC.

**As** can be seen from Figure 1, the dissolution profile of tablets prepared according to above composition and manufacturing procedure is comparable to that of the reference product.

### Example 9

| **Component** | **Amount [mg/tablet]** |
|---|---|
| Aliskiren hemifumarate (corresponding to 300mg of aliskiren) | 331,50 |
| Colloidal anhydrous hydrophobic silica (Aerosil R 972) | 7,00 |
| Talc | 40,00 |
| Powdered cellulose | 69,70 |
| Povidone K30 | 54,00 |
| Sodium hydroxide | 0,80 |
| Water | 85,00 |
| **Granulate (before drying)** | *503,00* |
| Xylitol for direct compression (Xylitab 200) | 132,00 |
| Crospovidone | 80,00 |
| Colloidal anhydrous silica (Aerosil 200 D) | 5,00 |
| Magnesium stearate | 20,00 |
| **Tablet core** | *740,00* |

The active ingredient was mixed with with colloidal anhydrous hydrophobic silica (Aerosil R972), talc, powdered cellulose and povidone in powder form. Then, the granulation liquid consisting of purified water and sodium hydroxide was sprayed onto the powder mixture for 5 minutes using a GPCG-1 fluid-bed granulator with top position of spray nozzle.

After drying and sieving, xylitol for direct compression (Xylitab 200), crospovidone and colloidal anhydrous silica (Aerosil 200 D) were added and mixed with the other components. Afterwards magnesium stearate was added as lubricant. Finally, the mixture was compressed into tablets. Preferably, the tablets are film coated using a standard water-based film coating using HPMC.

### Example 10

Comparison of stress stability of formulations according to present invention and reference 150 mg tablets (Rasilez batch No.: S0108) - tablets were packed in aluminum blisters and stored 28 days at 50°C (impurities were determined by HPLC chromatography)

| | Max. individual impurity (%) | Total impurities (%) |
|---|---|---|
| Reference | 0.18 | 0.72 |
| Example 2 | 0.15 | 0.57 |
| Example 4 | 0.17 | 0.62 |
| Example 5 | 0.13 | 0.51 |

### Example 11

Comparison of stress stability of formulations according to present invention and reference 300 mg tablets (Rasilez batch No.: S0086:)
- film coated tablets were packaged in aluminum blisters and stored 28 days at 40°C (impurities were determined by HPLC chromatography)

| | Max. individual impurity (%) | Total impurities (%) |
|---|---|---|
| Reference | 0.07 | 0.17 |
| Example 7 | 0.05 | 0.13 |
| Example 8 | 0.04 | 0.11 |
| Example 9 | 0.05 | 0.13 |

- film coated tablets were packaged in aluminum blisters (Example 8/1) or aluminum blisters with desiccant (Example 8/2) and stored 3 months at 40°C (impurities were determined by HPLC chromatography)

| | Max. individual impurity (%) | Total impurities (%) |
|---|---|---|
| Reference | 0.07 | 0.28 |
| Example 8/1 | 0.07 | 0.20 |
| Example 8/2 | 0.07 | 0.22 |

### Example 12

Dissolution profiles comparison of tablet cores of formulations according to present invention are shown in Table 1. Dissolution method: 1,000 ml of 0.1 M HC1 solution at 37 °C, apparatus 1(baskets) according to USP (US Pharmacopoeia) (average of six repetitions was used in the table 1).

**Table 1: Comparison of % of aliskiren dissolved from tablet cores prepared according to examples 2-5 of the present invention**

| Time (min)/ Batch No. | Ex. 2 (%) | Ex. 3 (%) | Ex.4 (%) | Ex.5 (%) |
|---|---|---|---|---|
| 0 | 0 | 0 | 0 | 0 |
| 5 | 40 | 29 | 28 | 30 |
| 10 | 65 | 55 | 48 | 52 |
| 15 | 87 | 73 | 65 | 71 |
| 20 | 102 | 86 | 82 | 88 |
| 30 | 104 | 96 | 103 | 102 |

### Example 13

### Influence of moisture content in the granulate on the stability of coated tablets.

In accordance with the procedure described in Example 7, granulates were prepared and dried to different levels of residual moisture.

In particular, the residual moisture in the granulate of Example 7/1 is 1.9 weight% and in the granulate of Example 7/2 1.4 weight%, as determined by halogen dryer (e.g. Mettler HR 73; 105°C, 5 minutes). The residual moisture in the film coated tablets of Example 7/1 is 3.8 weight% and in the film coated tablets of Example 7/2 3.4 weight%, as determined by halogen dryer (Mettler HR 73; 105°C, 5 minutes).

The table below shows a comparison of stress stability data of tablets prepared from the above granulates according to present invention (Example 7) with lower and higher moisture content - film coated tablets were packaged in aluminum blisters and stored 5 months at 40°C.

| Impurity | Example 7/1 t=0 (w/w%) | Example 7/1 Stored 5 months at 40°C (w/w% ) | Example 7/2 t=0 (w/w%) | Example 7/2 Stored 5 months at 40°C (w/w%) |
|---|---|---|---|---|
| impurity A (Rt=8.7min) | b.l.d. | 0.19 | b.l.d. | 0.13 |
| impurity B (Rt=29.8 min) | 0.01 | 0.21 | 0.02 | 0.11 |
| Total impurities | 0.03 | 0.63 | 0.03 | 0.39 |

| | | | | |
|---|---|---|---|---|
| R.t.: retention time; b.1.d.: below limit of detection **Impurity A:** 2-((3S,5S,6S,8S)-8-(3-amino-2,2-dimethyl-3-oxopropylcarbamoyl)-6-hydroxy-3-(4-methoxy-3-(3-methoxypropoxy)benzyl)-2,9-dimethyldecan-5-ylamino)succinic acid **Impurity B:** (3S,5S)-5-((1S,3S)-1-amino-3-(4-methoxy-3-(3-methoxypropoxy)benzyl)-4-methylpentyl)-3-isopropyldihydrofuran-2(3H)-one | | | | |

As can be seen from the above results, the stability of aliskiren in the tablets, expressed as the weight % (w/w%) of individual and total impurities, is dependent on the moisture content in the granulate and in the final (film coated) tablets, where lower moisture content in the granulate result in lower impurity content in the tablets and consequently better stability of the drug formulated into tablets upon storage.

The analytical method used to determine the impurities is characterized by the following features:
Analytical method: Column: ECLIPSE PLUS C-18; 150 mm x 4.6 mm i.d., 1.8 µm particle size
Mobile phase:
Gradient elution:
A: 0.01M ammonium acetate; pH-8.0
B: acetonitrile

| time (min) | 0 | 12 | 31 | 36 |
|---|---|---|---|---|
| % A | 72 | 60 | 20 | 20 |
| % B | 28 | 40 | 80 | 80 |

| | | | | |
|---|---|---|---|---|
| Post time = 8 min | | | | |

Flow: 0.5 ml/min
Injection: 10 µl
Detection: UV, 230 nm
Column temperature: 30°C
Solvent: acetonitrile : water = 30:70(V/V)

## Claims

1. A moisture-activated granulation process for the manufacture of a pharmaceutical dosage form containing at least one moisture-sensitive active substance selected from the group consisting of aliskiren and its pharmaceutically acceptable salts, esters and cocrystals, said process comprising:
a) mixing the active substance(s) with one or more dry excipients in solid form, said excipient being selected in particular from the group consisting of diluents, surfactants, binders, lubricants, disintegrators, granulation aids, buffering/alkalizing agents, fillers and antioxidants;
b) contacting the mixture with a granulation liquid consisting of water purified in accordance with pharmaceutical requirements and optionally one or more excipients to form a granulate, said excipient being selected in particular from the group consisting of binders, buffering/alkalizing agents, surfactants and antioxidants, wherein the contacting time is in the range of from 30 seconds to 5 minutes, and
wherein the ratio of the total amount of water to the total amount of all the solid ingredients including the active substance(s) and all intragranular excipients is less than 30:100, based on weight;
c) further processing the mixture including the addition of further dry excipients to obtain the pharmaceutical dosage form, said excipients being in particular selected from the group consisting of diluents, disintegrants, glidants and lubricants.

2. Process according to claim 1, wherein the ratio of the total amount of water to the total amount of all the solid ingredients including the active substance(s) and all intragranular excipients is less than 20:100, in particular less than 10:100, based on weight.

3. Process according to claim 1 or claim 2, wherein the one or more dry excipients mixed with the active ingredient in step (a) is selected from the group of granulation aids, diluents, lubricants, binders, fillers and optionally buffering/alkalizing agents.

4. Process according to claim 3, wherein the granulation aid is selected from substances having a surface area of more than 50 m²/g.

5. Process according to claim 3 or 4 wherein the granulation aid is selected from the group consisting of colloidal anhydrous silicas having a hydrophilic or hydrophobic surface and wherein the binder is selected from the group consisting of povidones having a K value of from 7 to 30 and wherein the lubricant is selected from the group consisting of talc and magnesium stearate.

6. Process according to any of the preceding claims, wherein the granulation liquid contains the purified water and optionally buffering/alkalizing agents selected from sodium or potassium hydroxide, and dibasic or tribasic sodium phosphate.

7. Process according to claim 6, wherein the granulation liquid additionally contains an excipient selected from the group consisting of binders, antioxidants and surfactants.

8. Process according to any of the preceding claims, wherein the active ingredient is aliskiren hemifumarate.

9. Process according to any of the preceding claims, wherein in step (c) an excipient selected from the group consisting of colloidal anhydrous silicas having a hydrophilic or hydrophobic surface and microcrystalline cellulose is added.

10. Process according to any of the preceding claims, wherein the pharmaceutical dosage form obtained is a dosage form suitable for oral delivery.

11. Process according to claim 10, wherein the dosage form is selected from the group consisting of tablets, film coated tablets, pills, lozenges, sachets, soft and hard gelatin capsules.

12. Process according to any of the preceding claims, wherein the granulate obtained after step (b) is dried so that the residual moisture in the granulate is kept below about 2 weight%, preferably below about 1.7 weight% and most preferably below about 1.5 weight%, as determined by a halogen dryer.

13. Process according to any of the preceding claims, wherein the pharmaceutical dosage form is present in the form of coated tablets which are dried so that the residual moisture in the tablet is kept below about 5 weight%, preferably below about 4.5 weight% and most preferably below about 3.8 weight%, as determined by a halogen dryer.

14. Pharmaceutical formulation obtainable by the process according to any of the preceding claims.

15. Pharmaceutical formulation according to claim 14 for use in the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache, chronic heart failure, erectile dysfunction, and female sexual dysfunction such as female arousal disorder.

## Patentansprüche

1. Feuchtigkeitsaktiviertes Granulierungsverfahren zur Herstellung einer pharmazeutischen Darreichungsform, die mindestens einen feuchtigkeitsempfindlichen Wirkstoff ausgewählt aus der Gruppe bestehend aus Aliskiren und seinen pharmazeutisch-verträglichen Salzen, Estern und Co-Kristallen enthält, bei welchem Verfahren:
a) der/die Wirkstoff(e) mit einem oder mehreren trockenen Hilfsstoffen in fester Form gemischt wird/werden, wobei der Hilfsstoff insbesondere ausgewählt ist aus der Gruppe bestehend aus Verdünnungsmitteln, Tensiden, Bindemitteln, Schmierstoffen, Desintegratoren, Granulierungshilfsmitteln, Puffern/alkalisch machenden Mitteln, Füllstoffen und Antioxidantien,
b) die Mischung mit einer Granulierungsflüssigkeit kontaktiert wird, die aus im Einklang mit pharmazeutischen Anforderungen gereinigtem Wasser und gegebenenfalls einem oder mehreren Hilfsstoffen besteht, so dass ein Granulat gebildet wird, wobei der Hilfsstoff insbesondere ausgewählt ist aus der Gruppe bestehend aus Bindemitteln, Puffern/alkalisch machenden Mitteln, Tensiden und Antioxidantien, wobei die Kontaktierungsdauer im Bereich von 30 Sekunden bis 5 Minuten liegt, und
wobei das Verhältnis der Gesamtmenge an Wasser zu der Gesamtmenge von allen festen Bestandteilen einschließlich des/der Wirkstoff(e) und allen intragranulären Hilfsstoffen weniger als 30:100 beträgt, bezogen auf Gewicht,
c) die Mischung weiterverarbeitet wird einschließlich der Zugabe weiterer trockener Hilfsstoffe, um die pharmazeutische Darreichungsform zu erhalten, wobei die Hilfsstoffe insbesondere ausgewählt sind aus der Gruppe bestehend aus Verdünnungsmitteln, Desintegratoren, Gleitmitteln und Schmierstoffen.

2. Verfahren nach Anspruch 1, bei dem das Verhältnis der Gesamtmenge an Wasser zur Gesamtmenge aller fester Bestandteile einschließlich des/der Wirkstoff(e) und aller intragranulärer Hilfsstoffe weniger als 20:100, insbesondere weniger als 10:100 beträgt, bezogen auf Gewicht.

3. Verfahren nach Anspruch 1 oder Anspruch 2, bei dem der eine oder die mehreren trockenen Hilfsstoffe, die in Stufe (a) mit dem aktiven Bestandteil gemischt werden, ausgewählt ist aus der Gruppe bestehend aus Granulierungshilfsmitteln, Verdünnungsmitteln, Schmierstoffen, Bindemitteln, Füllstoffen und gegebenenfalls Puffern/alkalisch machenden Mitteln.

4. Verfahren nach Anspruch 3, wobei das Granulierungshilfsmittel ausgewählt ist aus Substanzen, die eine Oberfläche von mehr als 50 m²/g aufweisen.

5. Verfahren nach Anspruch 3 oder 4, bei dem das Granulierungshilfsmittel ausgewählt ist aus der Gruppe bestehend aus kolloidalen wasserfreien Siliciumdioxiden, die eine hydrophile oder hydrophobe Oberfläche aufweisen, und bei dem das Bindemittel ausgewählt ist aus der Gruppe bestehend aus Povidonen, die einen K-Wert von 7 bis 30 aufweisen, und bei dem der Schmierstoff ausgewählt ist aus der Gruppe bestehend aus Talk und Magnesiumstearat.

6. Verfahren nach einem der vorhergehenden Ansprüche, bei dem die Granulierungsflüssigkeit das gereinigte Wasser und ggfs. Puffer/alkalisch machende Mittel ausgewählt aus Natrium- oder Kaliumhydroxid und zweibasischem oder dreibasischem Natriumphosphat enthält.

7. Verfahren nach Anspruch 6, bei dem die Granulierungsflüssigkeit zusätzlich einen Hilfsstoff ausgewählt aus der Gruppe bestehend aus Bindemitteln, Antioxidantien und Tensiden enthält.

8. Verfahren nach einem der vorhergehenden Ansprüche, bei dem der aktive Bestandteil Aliskirenhemifumarat ist.

9. Verfahren nach einem der vorhergehenden Ansprüche, bei dem in Stufe (c) ein Hilfsstoff ausgewählt aus der Gruppe bestehend aus kolloidalen nichtwässrigen Siliciumdioxiden, die eine hydrophile oder hydrophobe Oberfläche aufweisen, und mikrokristalliner Cellulose zugesetzt wird.

10. Verfahren nach einem der vorhergehenden Ansprüche, bei dem die pharmazeutische Darreichungsform eine Darreichungsform ist, die für orale Darreichung geeignet ist.

11. Verfahren nach Anspruch 10, bei dem die Verabreichungsform ausgewählt ist aus der Gruppe bestehend aus Tabletten, filmbeschichteten Tabletten, Pillen, Pastillen, Portionsbeuteln, weichen und harten Gelatinekapseln.

12. Verfahren nach einem der vorhergehenden Ansprüche, bei dem das nach Stufe (b) erhaltene Granulat getrocknet wird, so dass die Restfeuchtigkeit in dem Granulat unterhalb von 2 Gew.-% gehalten wird, vorzugsweise unterhalb von 1,7 Gew.-% und insbesondere unterhalb von etwa 1,5 Gew.-%, wie mittels eines Halogentrockners bestimmt.

13. Verfahren nach einem der vorhergehenden Ansprüche, bei dem die pharmazeutische Darreichungsform in Form von beschichteten Tabletten vorliegt, die so getrocknet werden, dass die Restfeuchtigkeit in der Tablette unterhalb von etwa 5 Gew.-%, und insbesondere unterhalb von etwa 3,8 Gew.-% gehalten wird, wie mittels eines Halogentrockners bestimmt.

14. Pharmazeutische Formulierung, die nach dem Verfahren gemäß einem der vorstehenden Ansprüche erhältlich ist.

15. Pharmazeutische Formulierung nach Anspruch 14 zur Verwendung bei der Behandlung von Bluthochdruck, kongestiver Herzinsuffizienz, Angina, Herzinfarkt, Arteriosklerose, diabetischen Nierenerkrankungen, diabetischer Herzmyopathie, Niereninsuffizienz, peripherer arteriosklerotischer Gefäßerkrankung, linksvendrikulärer Hypertrophie, kognitiver Dysfunktion, Schlaganfall, Kopfschmerz, chronischer Herzinsuffizienz, erektiler Dysfunktion und weiblicher sexueller Dysfunktion wie weiblicher Erregungsstörung (FSAD).

## Revendications

1. Procédé de granulation activée par humidité, servant à fabriquer une forme pharmaceutique contenant au moins un principe actif sensible à l'humidité, choisi dans l'ensemble formé par l'aliskirène et ses sels, esters et co-cristaux pharmacologiquement admissibles, lequel procédé comporte les opérations suivantes :
a) mélanger le ou les principe(s) actif(s), à l'état solide, avec un ou plusieurs excipient(s) sec(s), lequel ou lesquels excipient(s) est ou sont choisi(s) en particulier dans l'ensemble formé par les diluants, tensioactifs, liants, lubrifiants, désintégrants, auxiliaires de granulation, agents tampons/alcalinisants, charges et antioxydants ;
b) mettre le mélange en contact avec un liquide de granulation constitué d'eau, purifiée selon les exigences pharmaceutiques, et en option, d'un ou de plusieurs excipient(s), lequel ou lesquels excipient(s) est ou sont choisi(s) en particulier dans l'ensemble formé par les liants, agents tampons/alcalinisants, tensioactifs et antioxydants, de manière à former un granulat,
étant entendu que le temps de contact vaut de 30 secondes à 5 minutes et que le rapport de la quantité totale d'eau à la quantité totale de tous les ingrédients solides, y compris le ou les principe(s) actif(s) et tous les excipients intragranulaires, vaut moins de 30/100 en poids ;
c) et poursuivre le traitement du mélange, ce qui inclut l'addition d'autres excipients secs, lesquels excipients sont choisis en particulier dans l'ensemble formé par les diluants, désintégrants, agents de glissement et lubrifiants, pour obtenir la forme pharmaceutique.

2. Procédé conforme à la revendication 1, dans lequel le rapport de la quantité totale d'eau à la quantité totale de tous les ingrédients solides, y compris le ou les principe(s) actif(s) et tous les excipients intragranulaires, vaut moins de 20/100, et en particulier moins de 10/100, en poids.

3. Procédé conforme à la revendication 1 ou 2, dans lequel le ou les excipient(s) sec(s) mélangé(s) dans l'étape (a) avec le principe actif est ou sont choisi(s) dans l'ensemble formé par les auxiliaires de granulation, diluants, lubrifiants, liants et charges, et en option, agents tampons/alcalinisants.

4. Procédé conforme à la revendication 3, dans lequel l'auxiliaire de granulation est choisi parmi les substances qui présentent une aire spécifique de plus de 50 m²/g.

5. Procédé conforme à la revendication 3 ou 4, dans lequel l'auxiliaire de granulation est choisi dans l'ensemble formé par les silices anhydres colloïdales dotées d'une surface hydrophile ou hydrophobe, le liant est choisi dans l'ensemble constitué par les Povidones K7 à K30, et le lubrifiant est choisi parmi du talc et du stéarate de magnésium.

6. Procédé conforme à l'une des revendications précédentes, dans lequel le liquide de granulation contient de l'eau purifiée, et en option, des agents tampons/alcalinisants choisis parmi de l'hydroxyde de sodium, de l'hydroxyde de potassium, de l'hydrogénophosphate disodique et du phosphate trisodique.

7. Procédé conforme à la revendication 6, dans lequel le liquide de granulation contient en outre un excipient choisi dans l'ensemble formé par les liants, les antioxydants et les tensioactifs.

8. Procédé conforme à l'une des revendications précédentes, dans lequel le principe actif est de l'hémifumarate d'aliskirène.

9. Procédé conforme à l'une des revendications précédentes, dans lequel, dans l'étape (c), on ajoute un excipient choisi dans l'ensemble formé par la cellulose microcristalline et les silices anhydres colloïdales dotées d'une surface hydrophile ou hydrophobe.

10. Procédé conforme à l'une des revendications précédentes, dans lequel la forme pharmaceutique obtenue est une forme galénique appropriée pour administration par voie orale.

11. Procédé conforme à la revendication 10, dans lequel la forme galénique est choisie dans l'ensemble formé par les suivantes : comprimés, comprimés pelliculés, pilules, pastilles, sachets, et capsules de gélatine, molles ou dures.

12. Procédé conforme à l'une des revendications précédentes, dans lequel on fait sécher le granulat obtenu, après l'étape (b), de manière à ce que le taux d'humidité résiduelle dans le granulat, déterminé à l'aide d'un dispositif de séchage par lampe à halogène, soit abaissé et maintenu à moins d'environ 2 % en poids, de préférence, à moins d'environ 1,7 % en poids, et surtout, à moins d'environ 1,5 % en poids.

13. Procédé conforme à l'une des revendications précédentes, dans lequel la forme pharmaceutique se présente sous forme de comprimés enrobés qu'on fait sécher de manière à ce que le taux d'humidité résiduelle dans ces comprimés, déterminé à l'aide d'un dispositif de séchage par lampe à halogène, soit abaissé et maintenu à moins d'environ 5 % en poids, de préférence, à moins d'environ 4,5 % en poids, et surtout, à moins d'environ 3,8 % en poids.

14. Formulation pharmaceutique accessible par un procédé conforme à l'une des revendications précédentes.

15. Formulation pharmaceutique conforme à la revendication 14, pour utilisation dans le traitement d'une hypertension, d'une insuffisance cardiaque, d'une angine de poitrine, d'un infarctus du myocarde, d'une athérosclérose, d'une néphropathie diabétique, d'une cardiomyopathie diabétique, d'une insuffisance rénale, d'une maladie vasculaire périphérique, d'une hypertrophie du ventricule gauche, d'un dysfonctionnement cognitif, d'une attaque, de maux de tête, d'une insuffisance cardiaque chronique, d'un dysfonctionnement érectile, ou d'un dysfonctionnement sexuel féminin comme un trouble de l'excitation féminine.
